# EUROPÄISCHE PATENTANMELDUNG

(11) **EP 3 522 125 A1**
(43) Veröffentlichungstag der Anmeldung: **07.08.2019**
(21) Anmeldenummer: 19163966.5
(22) Anmeldetag: 16.10.2014
(51) Int. Cl.: G08B 13/196, G06K 9/00, A61B 5/11, A61B 5/00, A61B 5/107, G08B 21/02, G08B 21/22, G08B 25/00

(54) **VERFAHREN FÜR DIE ÜBERWACHUNG EINES PATIENTEN INNERHALB EINES MEDIZINISCHEN ÜBERWACHUNGSBEREICHS**

(30) Priorität: 17.10.2013 DE 102013017264
(62) Teilanmeldung aus: 14786607.3
(71) Anmelder: Drägerwerk AG & Co. KGaA, 23558 Lübeck (DE)
(72) Erfinder: Franz, Frank, 23617 Stockelsdorf (DE); Schlichting, Stefan, 23562 Lübeck (DE)

(57) **Zusammenfassung**

Die Erfindung betrifft ein Verfahren für die Überwachung eines Patienten (22a) innerhalb eines medizinischen Überwachungsbereichs (100) mittels eines Überwachungssystems (200) mit einer Tiefenkameravorrichtung (210), aufweisend die folgenden Schritte:
- Erzeugen einer Punktewolke (30) des Überwachungsbereichs (100) mit dem Überwachungssystem (200),
- Auswerten der Punktewolke (30) zur Erkennung vordefinierter Objekte (20), insbesondere Personen (22),
- Bestimmen des Ortes wenigstens eines erkannten Objekts (20) im Überwachungsbereich (100),
- Vergleich des bestimmten Ortes des wenigstens einen erkannten Objekts (20) mit wenigstens einem Vorgabewert (40) für den Ort dieses erkannten Objekts (20).

## Beschreibung

Die vorliegende Erfindung betrifft ein Verfahren für die Überwachung eines Patienten innerhalb eines medizinischen Überwachungsbereichs sowie ein Überwachungssystem für die Durchführung eines solchen Verfahrens.

Es ist grundsätzlich bekannt, dass Patienten sich in medizinischen Überwachungsbereichen einer Überwachung unterziehen müssen. Beispielsweise hängt es von der jeweiligen medizinischen Situation des Patienten ab, wie stark bzw. intensiv die Überwachung ausgebildet sein muss. So ist es beispielsweise problematisch, dass verwirrte Patienten häufig entgegen ärztlichen Anweisungen handeln. Beispielsweise können Patienten, welche mit einem Tubus versehen sind und einen verwirrten Geisteszustand aufweisen, dazu tendieren, sich den Tubus selbst zu entfernen. Darüber hinaus besteht das Risiko, dass Patienten, welche als bettlägerig eingestuft sind, das Bett verlassen und sich dabei verletzen. Auch besteht die Gefahr von Krampfanfällen, sodass bei einem Krampfanfall zusätzlich medizinische Beeinträchtigungen für den Patienten entstehen können. Um die voranstehenden Risiken in den Griff zu bekommen bzw. zu reduzieren, ist ein außerordentlich hoher Personalaufwand notwendig. So ist beispielsweise eine persönliche Überwachung mithilfe von Überwachungspersonal notwendig, sodass im Extremfall die überwachende Person sich die gesamte Zeit im Inneren des Patientenzimmers befinden muss. Dies ist mit sehr hohem Kostenaufwand versehen, sodass dies nur in den seltensten Fällen tatsächlich gewährleistet ist. Eine andere bekannte Möglichkeit ist das Verwenden von Überwachungskameras, sodass in einem Monitorraum eine entsprechende Überwachungsperson mehrere Patienten gleichzeitig mithilfe der Überwachungsmonitore überwachen kann. Auch hier ist jedoch ein immer noch relativ hoher Personaleinsatz notwendig, da diese Überwachung üblicherweise lückenlos 24 Stunden und 7 Tage die Woche vollzogen werden muss. Bei der Überwachung mehrerer Patienten durch eine Überwachungsperson ist darüber hinaus deren Aufmerksamkeit für die tatsächliche Risikoeinstufung durch die Überwachung entscheidend. Insbesondere Unaufmerksamkeit oder Schläfrigkeit der Überwachungsperson führen zu nicht kalkulierbarem Anstieg des Risikos für den Patienten. Ein weiterer Nachteil besteht in der Beeinträchtigung der Privatsphäre des Patienten, von Angehörigen oder von Mitarbeitern.

Es ist Aufgabe der vorliegenden Erfindung, die voranstehend beschriebenen Nachteile zumindest teilweise zu beheben. Insbesondere ist es Aufgabe der vorliegenden Erfindung, in kostengünstiger und einfacher Weise wenigstens zum Teil eine Automatisierung der Überwachung der Patienten zu erreichen und bevorzugt auch eine zumindest teilweise Automation der Dokumentation und der Bereitstellung von Kontextinformationen für andere medizinische Geräte zu ermöglichen.

Voranstehende Aufgabe wird gelöst durch ein Verfahren mit den Merkmalen des Anspruchs 1 und ein Überwachungssystem mit den Merkmalen des Anspruchs 30. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit dem erfindungsgemäßen Überwachungssystem und jeweils umgekehrt, sodass bezüglich der Offenbarung zu den einzelnen Erfindungsaspekten stets wechselseitig Bezug genommen wird bzw. werden kann.

Ein erfindungsgemäßes Verfahren dient der Überwachung eines Patienten innerhalb eines medizinischen Überwachungsbereichs mittels eines Überwachungssystems mit einer Tiefenkameravorrichtung. Hierfür weist das erfindungsgemäße Verfahren die folgenden Schritte auf:
- Erzeugen einer Punktewolke des Überwachungsbereichs mit dem Überwachungssystem,
- Auswerten der Punktewolke zur Erkennung vordefinierter Objekte, insbesondere Personen,
- Bestimmen des Ortes wenigstens eines erkannten Objekts im Überwachungsbereich,
- Vergleich des bestimmten Ortes des wenigstens einen erkannten Objekts mit wenigstens einem Vorgabewert für den Ort dieses erkannten Objekts.

Der Schritt des Vergleichs in Form einer Umwelteingabe aktiviert wenigstens eine Funktionalität des Verfahrens.

Erfindungsgemäß erfolgt also wenigstens teilweise eine automatisierte Durchführung eines Überwachungsverfahrens. Dabei wird zurückgegriffen auf Dateninformationen des Überwachungssystems. Dieses Überwachungssystem ist mit einer Tiefenkameravorrichtung ausgestattet, welche in der Lage ist, Tiefeninformationen des Überwachungsbereichs zur Verfügung zu stellen. Dabei kann es sich z. B. um eine Tiefenkamera oder eine Kombination mehrerer Tiefenkameras handeln. Selbstverständlich kann eine erfindungsgemäße Tiefenkameravorrichtung bzw. das Überwachungssystem auch noch über zusätzliche weitere und vor allem anders geartete Sensoren verfügen. Der Kern ist jedoch die Möglichkeit, eine Punktewolke zu erzeugen, wofür die Tiefeninformationen der Tiefenkameravorrichtung in kostengünstiger und einfacher Weise zur Verfügung gestellt werden können.

Unter dem medizinischen Überwachungsbereich ist insbesondere ein Patientenzimmer in einem Krankenhaus zu verstehen. Jedoch sind auch andere medizinische Einrichtungen mit einem erfindungsgemäßen Verfahren überwachbar. So können ganze Krankenhaustrakte, die Flure und Treppenhäuser des Krankenhauses sowie ganze Krankenhausflügel durch ein erfindungsgemäßes Verfahren überwacht werden. Der Kern der vorliegenden Erfindung und auch die hauptsächlichen Vorteile werden jedoch insbesondere bei lokal beschränkten Überwachungsbereichen, wie z. B. einem Flur in einem Krankenhaus bzw. einzelnen Patientenzimmern, erreicht.

Die Tiefenkameravorrichtung ist in der Lage Tiefeninformationen zur Verfügung zu stellen.

Je nach Qualität der jeweiligen Tiefenkamera geschieht dies mit unterschiedlichen Auflösungen. Das Ergebnis einer solchen Überwachung mit einer Tiefenkameravorrichtung ist üblicherweise eine dreidimensionale Punktewolke. Dabei können einzelne Punkte voneinander unterschieden werden hinsichtlich ihrer Qualität und hinsichtlich ihrer örtlichen Darstellung. So können Abstände einzelner Punkte zueinander im Raum in dreidimensionaler Weise festgestellt werden. Werden solche dreidimensionalen Bilder über einen längeren Zeitraum hin aufgenommen, entsteht dementsprechend eine vierdimensionale Informationsstruktur, aus welcher die Veränderung der Punktewolke als vierte Dimension erkennbar wird. Dabei kann das Überwachungssystem mit der Tiefenkameravorrichtung im Zusammenspiel die Auswertung vornehmen. Entscheidend ist, dass die Punktewolke als Ergebnis der Tiefenkameravorrichtung sozusagen ein Szenario des Überwachungsraums darstellt. Die Interpretation dieses Szenarios kann sowohl noch in der Tiefenkameravorrichtung als auch erst im Überwachungssystem erfolgen. Dabei sind zwei wesentliche Schritte zu unterscheiden. Der erste Schritt ist das grundsätzliche Erstellen eines Szenarios, zu welchem die beiden mittleren Schritte des erfindungsgemäßen Verfahrens dienen. So wird in einem ersten Schritt dieses ersten Teils ein Auswerten der Punktewolke dahingehend erfolgen, dass die Erkennung vordefinierter Objekte erfolgt. Vordefinierte Objekte sind unterschiedliche Formen bzw. unterschiedliche Konturen oder unterschiedliche Volumina, welche in der Punktewolke als solche separat erkennbar sind. So können einzelne Objekte, wie z. B. Tische, Stühle oder Betten wie auch und besonders bevorzugt Personen, erkannt werden. Die Erkennung erfolgt durch die Vordefinition dieser Objekte mit entsprechenden objektspezifischen Parametern. Dies wird später noch näher erläutert.

Der zweite Schritt dieses Teils des Verfahrens dient dazu, über die Tiefeninformationen der Punktewolke eine exakte Ortsbestimmung des erkannten Objekts im Überwachungsbereich durchzuführen. Hier handelt es sich in einem ersten Schritt um die dreidimensionale Positionierung unabhängig von der Zeit auf Basis einer dreidimensionalen Punktewolke. Im Ergebnis dieser beiden Schritte des Teils des Verfahrens entsteht also ein Szenario, in welchem Informationen über erkannte vordefinierte Objekte einerseits und deren explizite Orte im Überwachungsbereich andererseits bestehen. Diese erste Auswertung der Informationen wird im Sinne der vorliegenden Erfindung auch als Szenario bezeichnet, welches als Ergebnis dieser beiden Auswertungs- und Bestimmungsschritte steht.

Anschließend an das Erstellen eines Szenarios können unterschiedlichste Funktionalitäten auf Basis dieses Szenarios ausgearbeitet werden. Die Auswertung des Szenarios kann auch als einzelne Überwachungsfunktion eines erfindungsgemäßen Verfahrens verstanden werden. Die Gemeinsamkeit für sämtliche unterschiedlichen Funktionen eines erfindungsgemäßen Verfahrens ist die Durchführung eines grundlegenden Schrittes. Dabei handelt es sich um den Vergleich des bestimmten Ortes des wenigstens einen erkannten Objekts mit wenigstens einem Vorgabewert für den Ort dieses erkannten Objekts. Dabei spielen mehrere Parameter mit. So ist für unterschiedliche Objekte eine unterschiedliche Vorgabe für den jeweiligen gewünschten Ort dieses erkannten Objekts definierbar. Mit anderen Worten sind die Vorgabewerte dementsprechend ebenfalls objektspezifisch. Der Vorgabewert beinhaltet also eine Ortsposition, an welcher sich dieses Objekt wunschgemäß befinden soll. Damit wird durch den Vergleich auch eine Abweichung des tatsächlich erkannten bzw. bestimmten Ortes des erkannten Objekts von dem gewünschten Vorgabewert für diesen Ort erkennbar. Diese Beabstandung kann zur Überwachung für unterschiedlichste Funktionen herangezogen werden. Der einfachste und anschaulichste Fall ist dabei eine Funktion des sogenannten "Virtual Fence", also einem virtuellen Zaun bzw. ein virtuelles Bettgitter. Damit kann als Vorgabewert für den Ort eines erkannten Objekts ein expliziter Ort oder eine Ortsmenge vorgegeben werden, an welchen sich das Objekt befinden darf bzw. nicht befinden darf. Die Grenze zwischen den Orten, welche als zulässig und welche als unzulässig für das jeweilige Objekt definiert sind, kann als virtueller Zaun bezeichnet werden. Mit einem erfindungsgemäßen Verfahren kann das Szenario also dahingehend ausgewertet werden, ob sich das Objekt innerhalb oder außerhalb des virtuellen Zauns befindet. Handelt es sich bei dem Objekt um eine als Patient erkannte Person, so führt dies dazu, dass eine Erlaubnisstruktur für diesen Patienten vorgegeben werden kann. Darf dieser Patient aus medizinischen und Sicherheitsgründen sein Patientenzimmer und/oder sein Bett nicht verlassen, so kann ein solcher virtueller Zaun diese Überwachung gewährleisten. Befindet sich der Patient als Objekt innerhalb dieses virtuellen Zauns, ist für das Verfahren kein Grund für eine Alarmierung von Bediensteten des Krankenhauses gegeben. Bewegt sich das Objekt in Form des Patienten auf den virtuellen Zaun zu und überschreitet er diesen sogar im Ganzen oder mit einzelnen Körperteilen, so kann eine Alarmierung erfolgen, sodass entsprechend sofort Aktionen für das Zurückbringen des Patienten in sein Patientenzimmer eingeleitet werden können. Bei der voranstehend beschriebenen Funktionalität handelt es sich um eine von unterschiedlichen Möglichkeiten zur Auswertung eines zur Verfügung gestellten Szenarios. Dabei wird besonders deutlich, dass unterschiedlichste Funktionalitäten auf dem gleichen Szenario aufbauen können, sodass einmal zur Verfügung gestellte Daten aus der Punktewolke und deren Auswertung in Form des Szenarios mehrfach für unterschiedliche Überwachungsfunktionen Verwendung finden können. Auch wird hier bereits ersichtlich, dass die einzelnen Überwachungsfunktionen aus demselben Grund ohne Weiteres miteinander kombinierbar sind, ohne zusätzlichen Aufwand für die Datenerzeugung zu benötigen.

Die voranstehende Erläuterung der einzelnen Schritte wird über die Zeit wiederholt ausgeführt. So wird sowohl der Schritt der Szenarioerstellung in vierdimensionaler Weise durchgeführt als auch die Schritte des Überwachens durch den Vergleich. Mit anderen Worten entsteht damit eine kontinuierliche Durchführung eines erfindungsgemäßen Verfahrens über die Zeit, sodass sozusagen eine vierdimensionale Überwachung für die einzelnen Überwachungsfunktionen möglich ist. Bezogen auf den jeweiligen Ort des Objekts wird nach dem Erkennen des Objekts in einem Szenario sozusagen ein "Tracking Prozess" bzw. ein Nachverfolgungsprozess durchgeführt, also das Objekt hinsichtlich seines Ortes innerhalb des Überwachungsbereichs verfolgt.

Der Schritt des Auswertens der Punktewolke kann dabei unterschiedlichste objektspezifische Informationen in Betracht ziehen. Dazu können z. B. Farbinformationen der einzelnen Punkte der Punktewolke dienen. Auch typische Konturen, welche aus der Punktewolke erkennbar sind, können auf entsprechende Objekte, z. B. einen Tisch oder einen Stuhl bzw. eine Person, hinweisen. Auch ein erkanntes zu erwartendes Volumen in qualitativer und/oder quantitativer Weise kann zur objektspezifischen Auswertung der Punktewolke herangezogen werden. Auch der tatsächliche Ort des Objekts, z. B. stehend auf dem Boden, kann als Information für die Auswertung dienen. Nicht zuletzt ist auch die zeitliche Änderung der jeweiligen zugehörigen Punkte in der Punktewolke eine Informationsquelle für die Auswertung hinsichtlich vordefinierter Objekte. Insbesondere können auf diese Weise besonders schnell und einfach statische von bewegten Objekten unterschieden werden.

Es kann von Vorteil sein, wenn bei einem erfindungsgemäßen Verfahren die Auswertung der Punktewolke bzw. eines Teils der Punktewolke zur Erkennung wenigstens eines vordefinierten Objekts anhand vordefinierter objektspezifischer Parameter erfolgt, insbesondere wenigstens einer der folgenden:
- Ort der Punktewolke,
- Zeitliche Änderung des Ortes der Punktewolke,
- Kontur der Punktewolke,
- Zeitliche Änderung der Kontur der Punktewolke,
- Volumen der Punktewolke,
- Zeitliche Änderung des Volumens der Punktewolke.

Bei der voranstehenden Aufzählung handelt es sich um eine nicht abschließende Liste.

Insbesondere wird nochmals auf die zeitliche Änderung der jeweiligen Parameter hingewiesen, sodass damit auch ein Bewegungsprofil des jeweiligen Objekts für die Erkennung und die dementsprechende Vordefinition herangezogen werden kann. Die Orte der Punktewolke bzw. die Orte des Objekts sind dabei in dreidimensionaler Weise als Tiefeninformation zu verstehen. Bei der Kontur handelt es sich sozusagen um eine zweidimensionale Wiedergabe als Projektion der dreidimensionalen Information der Tiefenkameravorrichtung. Ein Volumen ist ein abgeschätztes Volumen auf Basis der wiederum nun dreidimensional zur Verfügung gestellten Informationen der Tiefenkameravorrichtung. Selbstverständlich können auch andere Informationen, z. B. aus weiteren Sensorvorrichtungen der Tiefenkameravorrichtung oder des Überwachungssystems zur Verfügung gestellt werden, um eine entsprechende Auswertung zur Erkennung wenigstens eines vordefinierten Objekts durchzuführen. Selbstverständlich kann auch eine Kombination unterschiedlicher objektspezifischer Parameter die Genauigkeit bei der Erkennung vordefinierter Objekte und insbesondere bei der Unterscheidung ähnlicher vordefinierter Objekte erhöhen. Das Volumen der Punktewolke kann dabei auch durch Teile von Objekten begrenzt werden, so dass sich dieses Volumen als Raum, welcher durch die Punktewolke umschlossen wird, ergibt.

Ein weiterer Vorteil ist erzielbar, wenn bei einem erfindungsgemäßen Verfahren ein als Person erkanntes Objekt anhand wenigstens eines personenspezifischen Parameters als Patient erkannt wird, insbesondere anhand wenigstens eines der folgenden personenspezifischen Parameter:
- Ort der Person,
- Explizite Markierung durch eine dritte Person, z.B. durch die noch beschriebene Umwelteingabe
- Eigenschaften der Kontur der Person.

Insbesondere wird dabei auch auf die zeitliche Änderung des jeweiligen Parameters abgestellt. So kann eine Person durch ein Bewegungsprofil noch relativ schnell bzw. einfach erkannt werden. Jedoch ist die Unterscheidung unterschiedlicher Personen und insbesondere die Definition einer Person als Patient von entscheidender Bedeutung bei der Überwachung, insbesondere um unnötige Fehlalarme zu vermeiden. Diese Definition erfolgt vorzugsweise in initialer Ausbildung, sodass eine einmal durchgeführte Definition einer Person als Patient durch Tracking dieses Patienten beibehalten wird. Dies gilt auch dann, wenn die als Patient definierte Person möglicherweise Bewegungen innerhalb des Überwachungsbereichs durchführt, welche nun nicht mehr vollständig die für den Patienten spezifischen Parameter erfüllt. Dies kann als Initialisierung eines Objekts in Form einer Person als Patient bezeichnet werden. Dabei kann z. B. das Einsteigen einer Person in das Bett in einem Überwachungsbereich als initiale Definition dieser Person als Patient beschrieben werden. Selbstverständlich sind auch andere Möglichkeiten, wie z. B. das Erkennen einer Intubierung dieser Person, für die Definition als Patient denkbar. Um einen Tubus zu erkennen, kann beispielsweise eine Verbindung zu benachbarten medizinischen Geräten, insbesondere in Form eines Beatmungsgeräts bzw. eine Schlauchverfolgung zu dem Tubus, Verwendung finden. Auch hierzu erfolgt später noch eine nähere Erläuterung.

Weiter kann es vorteilhaft sein, wenn bei einem erfindungsgemäßen Verfahren bei einer Überschreitung einer vordefinierten Abweichung von dem wenigstens einem Vorgabewert für den Ort des erkannten Objekts ein Alarmsignal ausgegeben wird. Unter einem Alarmsignal ist im Sinne der vorliegenden Erfindung eine bewusste und definierte Informationsgebung an ein medizinisches Personal zu verstehen. So kann auch hier wieder ein medizinisches Personal eines Krankenhauses in einem Überwachungsraum angeordnet sein, in welchem dieser Alarm, z. B. in Form eines Schwesternnotrufs, ausgegeben wird. Auch ist es möglich, diesen Alarm direkt an medizinisches Personal in Form von Ärzten auf deren Pager oder deren Mobiltelefon weiterzuleiten. Damit wird deutlich, dass die tatsächliche Überwachungsfunktionalität im Wesentlichen sogar vollständig automatisch zur Verfügung gestellt wird. Erst im Falle eines erkannten Überwachungsfalls, wenn also Alarm gegeben wird, muss der tatsächliche Eingriff durch medizinisches Personal erfolgen. Das Alarmsignal kann dabei aufgeteilt sein in unterschiedliche Alarmstufen. So ist es mit Bezug auf die beschriebene Funktionalität eines virtuellen Zauns möglich, unterschiedliche Zäune in einem Überwachungsraum zu definieren, sodass ein Voralarm von einem Hauptalarm unterscheidbar wird. So kann beispielsweise das Aufstehen eines Patienten aus seinem Bett einen Voralarm auslösen, während das Verlassen des Zimmers durch die Zimmertür den Hauptalarm auslöst. Diese abgestufte Alarmierung kann auch unterschiedliche Alarmwege zur Folge haben, sodass z. B. beim Verlassen des Zimmers nicht nur das medizinische Personal, sondern auch ein Sicherheitsdienst informiert wird.

Nachfolgend werden unterschiedliche Überwachungsfunktionen eines erfindungsgemäßen Verfahrens näher erläutert. Dabei handelt es sich im Wesentlichen ausschließlich um den zweiten Teil des erfindungsgemäßen Verfahrens, also das Auswerten des jeweiligen Szenarios. Bereits an dieser Stelle ist darauf hinzuweisen, dass durch die Verwendung eines gemeinsamen Szenarios aus den ersten beiden Schritten des erfindungsgemäßen Verfahrens, nämlich dem Auswerten der Punktewolke und dem Bestimmen des Ortes, immer das gleiche Szenario zur Verfügung gestellt wird. Dieses Szenario wird durch die unterschiedlichen Überwachungsfunktionen gemeinsam, jedoch mit unterschiedlichen Ergebnissen und unterschiedlichen Zielrichtungen verwendet. Des Weiteren unterscheiden sich die nachfolgend beschriebenen Überwachungsfunktionen durch im Wesentlichen einen entscheidenden Punkt. So ist für die nachfolgende Auswertung von entscheidender Bedeutung, ob der Vorgabewert einen Bezugspunkt innerhalb eines weiteren Objekts oder in absoluter Weise im Überwachungsbereich aufweist. Ein Bezugspunkt im Überwachungsbereich kann auch als absoluter Vorgabewert verstanden werden. Ein Bezugspunkt an einem weiteren Objekt kann als relativer Vorgabewert verstanden werden. Dabei ist darauf hinzuweisen, dass ein Objekt selbstverständlich einzelne Teilobjekte aufweisen kann. Besonders gut ersichtlich ist dies bei einem Objekt in Form einer Person, welches einzelne Objekte durch Teilobjekte durch die Körperglieder, Gelenkpunkte oder andere Körperteile aufweisen kann. So können sich einzelne Objekte selbstverständlich überschneiden bzw. ein großes Objekt aus mehreren kleinen Objekten zusammengesetzt sein.

Wie im voranstehenden Absatz erläutert worden ist, kann es von Vorteil sein, wenn bei einem erfindungsgemäßen Verfahren der wenigstens eine Vorgabewert für den Ort des erkannten Objekts als absoluter Vorgabewert bezogen auf den Überwachungsbereich ausgebildet ist. Darunter ist zu verstehen, dass als Vorgabewert eine absolute Ortsdefinition für den Ort des erkannten Objekts bezogen auf den Überwachungsbereich angegeben wird. Eine Relation zu anderen Objekten besteht in diesem Fall nicht oder wenn, dann nur zusätzlich zu diesem absoluten Vorgabewert. Damit wird eine besonders einfache, kostengünstige und vor allem mit geringem Rechenaufwand verbundene Auswertung eines zur Verfügung gestellten Szenarios möglich.

Eine mögliche Überwachungsfunktionalität mit einem absoluten Vorgabewert ist im Sinne der vorliegenden Erfindung der sogenannte virtuelle Zaun. Dieser ist selbstverständlich mit sämtlichen anderen Überwachungsfunktionalitäten der vorliegenden Erfindung frei kombinierbar. Auch kann er in Kombination sowohl absolute als auch relative Vorgabewerte in sich vereinen.

Es kann also von Vorteil sein, wenn bei einem erfindungsgemäßen Verfahren es sich bei dem wenigstens einen absoluten Vorgabewert um eine Ortsgrenze handelt, welche das erkannte Objekt, insbesondere eine als Patient definierte Person, nicht überschreiten darf. Dieser absolute Vorgabewert kann selbstverständlich auch durch ein Ergebnis der Auswertung der Punktewolke zur Verfügung gestellt werden. Wie bereits in der Einleitung erläutert, wird ein solcher virtueller Zaun als Ortsgrenze definiert, welcher z. B. um ein Bett herum definiert ist. Bewegt sich die Person über diese Ortsgrenze hinweg, so kann ein entsprechender Alarm gegeben werden. Auch können hier unterschiedliche Ortsgrenzen mit unterschiedlichen Alarmstufen, also z. B. einem Voralarm und einem Hauptalarm, gekoppelt sein. Damit wird sichergestellt, dass bettlägerige Personen ihr Bett nicht verlassen können, ohne medizinisches Fachpersonal darauf aufmerksam zu machen. Somit erfolgt sozusagen eine Sicherheit gegen den Ausbruch der Patienten aus dem Bett bzw. aus dem Zimmer im Überwachungsbereich. Dabei ist darauf hinzuweisen, dass diese als Ortsgrenze ausgebildete Version eines absoluten Vorgabewerts selbstverständlich auch eine dreidimensionale Erstreckung aufweisen kann. So kann auch oberhalb des Bettes eine Ortsgrenze definiert werden, welche beim Aufrichten des Patienten im Bett durch dessen Oberkörper durchbrochen wird. Somit wird bereits das Aufrichten im Bett als Überschreiten eines absoluten Vorgabewerts erkannt und ein entsprechendes Alarmsignal kann ausgegeben werden. Für einen Hauptalarm kann eine zusätzliche Ortsgrenze im Bereich der Tür des Patientenzimmers im Überwachungsbereich definiert werden, um den Hauptalarm beim Ausbruch aus dem Zimmer auslösen zu können.

Ein Verfahren gemäß dem voranstehenden Absatz kann dahingehend weitergebildet werden, dass bei einer erstmaligen Bewegung des erkannten Objekts in Form einer Person über die Ortsgrenze diese Person als Patient definiert wird. Wie ebenfalls bereits erläutert worden ist, ist es ein Vorteil der vorliegenden Erfindung, dass einzelnen Objekten in Form von Personen zusätzliche Informationen zugeordnet werden können. So können Personen z. B. als Patienten initial definiert werden und bleiben auch durch das Tracking der Orte dieser Person als Patient definiert über den Zeitraum der Überwachung. Beispielsweise kann eine Ortsgrenze um das Bett in einem Patientenzimmer im Überwachungsbereich gelegt sein. Überschreitet eine Person diese Ortsgrenze und legt sich dementsprechend in das Bett im Patientenzimmer, so ist mit hoher Wahrscheinlichkeit davon auszugehen, dass diese Person ein Patient ist. Selbstverständlich kann auch noch eine Kopplung mit einer Mindestzeitdauer für das Überschreiten der Ortsgrenze erfolgen, um mit höherer Sicherheit die richtige Person auch als Patient zu definieren. Auch das Einfahren eines belegten Bettes in ein Patientenzimmer kann zur Definition der darin befindlichen Person als Patient dienen.

Ein weiterer Vorteil wird erzielt, wenn bei einem erfindungsgemäßen Verfahren gemäß den beiden voranstehenden Absätzen die Schritte des Verfahrens nur für den Ortsabschnitt des Überwachungsbereichs auf der Seite der Ortsgrenze durchgeführt werden, auf welcher sich das erkannte Objekt befindet. Auch hierbei handelt es sich um eine Zusatzfunktionalität der Überwachungsfunktion "virtueller Zaun". Es wird auf diese Weise eine Reduktion des tatsächlichen überwachten Abschnitts des Überwachungsbereichs möglich, sodass dementsprechend eine Reduktion der notwendigen Datenmengen und der entsprechenden Auswertung erfolgen kann. Wird beispielsweise ein innerer Bereich im Überwachungsbereich auf das Bett definiert, so kann bei einem Patienten, welcher sich in das Bett gelegt hat, die nachfolgende Überwachung dieses Patienten sich auf den Bereich innerhalb der Ortsgrenze um das Bett beschränken. Die Reduktion der Datenmenge kann sich dabei zum einen auf die Erzeugung der Punktewolke und zum anderen auf deren Auswertung zur Erstellung eines Szenarios beziehen.

Darüber hinaus ist eine weitere Überwachungsfunktionalität bei einer erfindungsgemäßen Ausbildung des Verfahrens möglich. So kann eine Sedierungsüberwachung durchgeführt werden, welche die Sedierung und damit die Ruhigstellung eines Patienten überwacht.

Auch hierbei handelt es sich bevorzugt um die Nutzung absoluter Vorgabewerte im Sinne der vorliegenden Erfindung.

So kann es bereits von Vorteil sein, wenn bei einem erfindungsgemäßen Verfahren bei dem wenigstens ein absoluter Vorgabewert um eine maximale und/oder minimale Veränderung des Ortes des erkannten Objekts über die Zeit handelt. Mit anderen Worten wird bevorzugt sogar der Gradient der Änderung über die Zeit überwacht, sodass ein Aktivitätsindex erstellt werden kann, welcher mit der tatsächlichen Aktivität des Patienten korreliert. Dabei kann der jeweilige Patient im Gesamten oder einzelne Körperglieder als Teilobjekte der Person überwacht werden. Der Aktivitätsindex dient also dazu, die tatsächliche Aktivität einzelner Körperteile zu erkennen und damit einen Rückschluss auf die aktuelle Aktivitätssituation des Patienten zu ziehen. Damit wird es möglich, dass die aktuelle Aktivität in Bezug gesetzt wird zur gewünschten Sedierung. Ist ein Patient mit starker Sedierung versehen, so wird dementsprechend auch von einer geringeren Aktivität ausgegangen. Ist jedoch dem entgegengesetzt eine höhere Aktivität, also eine stärkere Veränderung der Orte des Objekts über die Zeit, als vorgegeben erkennbar, so muss es sich mit hoher Wahrscheinlichkeit um eine zu geringe Dosierung der Sedierung handeln.

Auch hier kann wieder eine entsprechende Ausgabe eines Alarms erfolgen. Dabei kann z. B. das Erkennen einzelner Skelettpunkte einer Person als Teilobjekte dieser Person in der Punktewolke erfolgen. Auf diese Weise wird noch genauer und detaillierter der Aktivitätsindex und damit die Erkennung der Sedierungsqualität möglich. Selbstverständlich kann über den Aktivitätsindex auch in entgegengesetzter Weise eine sogenannte Deliriumüberwachungsfunktionalität zur Verfügung gestellt werden. So wird es hier möglich, eine Person, welche in unerwünschter Weise zu ruhig wird, also z. B. in einen komatösen Zustand fällt, zu erkennen. Hier wird gut ersichtlich, dass die Überwachungsfunktionalität der Sedierungsüberwachung und die Überwachungsfunktionalität der Deliriumüberwachung vorzugsweise miteinander kombiniert eingesetzt werden. Hier können beliebig viele Grenzen gesetzt werden, um einen Alarm in der jeweiligen Richtung, also z. B. der ausreichenden Sedierung und der Deliriumüberwachung zur Verfügung zu stellen.

Bei der Sedierungsüberwachung gemäß dem voranstehenden Absatz kann es weiter von Vorteil sein, wenn bei dem erfindungsgemäßen Verfahren es sich bei dem vordefinierten und erkannten Objekt um wenigstens ein Körperteil einer Person handelt. Insbesondere ist hier davon auszugehen, dass die Extremitäten der Person in Form der Finger, der Hände, der Arme oder der Beine den besten Aussagewert hinsichtlich des aktuellen Aktivitätsindex darstellen. Auch können z. B. bei entsprechender Auflösung der Tiefenkameravorrichtung die Augenlieder der Person überwacht werden. Damit wird eine noch weitere Verbesserung einer Sedierungsüberwachung als Überwachungsfunktionalität eines erfindungsgemäßen Verfahrens möglich.

Bei der Sedierungsüberwachung als Funktionalität eines erfindungsgemäßen Verfahrens kann es darüber hinaus von Vorteil sein, wenn der absolute Vorgabewert in Form der maximalen und/oder minimalen Veränderung des Ortes des erkannten Objekts einstellbar ausgebildet ist. So kann eine Sedierung eines Patienten in unterschiedlichen Quantitäten erfolgen. Beispielsweise kann eine Person komplett ruhiggestellt werden, sodass davon auszugehen ist, dass im Wesentlichen keinerlei Aktivität mehr vorliegt. Auch kann eine nur leichte Sedierung eine Beruhigung eines Patienten zur Verfügung stellen, welcher ansonsten nur leicht verlangsamte Aktivität aufweist. Um die Überwachung zu verbessern und Fehlalarme zu vermeiden, kann eine Einstellung des Aktivitätsindex oder sogar direkt der jeweiligen Vorgabewerte für die entsprechende maximale oder minimale Veränderung des Ortes des erkannten Objekts erfolgen.

Das Einstellen der Überwachungsfunktionalität kann manuell oder durch sogenannte Umwelteingabe erfolgen. Unter einer Umwelteingabe ist im Sinne der vorliegenden Erfindung eine aktive Einschaltung der jeweiligen Überwachungsfunktionalität durch das erfindungsgemäße Verfahren selbst zu verstehen. Wird beispielsweise in dem Überwachungsbereich ein Beatmungsgerät erkannt, so wird eine entsprechende Überwachung der Beatmung durchgeführt. Wird beispielsweise eine Sediervorrichtung durch ein erfindungsgemäßes Verfahren als Objekt erkannt, so kann die voranstehende beschriebene Überwachungsfunktionalität der Sedierungsüberwachung durchgeführt werden. Die Umwelteingabe kann auch z.B. durch die Neigung des Bettes oder die Stellung eines Bettgitters erfolgen.

Bei einer Überwachungsfunktionalität in Form einer Sedierungsüberwachung ist es weiter vorteilhaft, wenn bei dem erfindungsgemäßen Verfahren die Veränderung des Ortes des erkannten Objekts über die Zeit mittels der quadratischen Abweichung des bestimmten Ortes des erkannten Objekts erfolgt. Dabei wird zur Reduktion der Fehleranfälligkeit sozusagen ein Ausmitteln der Überwachung durchgeführt. Insbesondere bezieht sich dabei diese quadratische Abweichung auf die jeweiligen Orte von Skelettpositionen bzw. Körpergliedpositionen der Person.

Um eine weitere Überwachungsfunktionalität beim erfindungsgemäßen Verfahren zur Verfügung zu stellen, kann beispielsweise eine Krampfüberwachung durchgeführt werden. Hierfür kann ebenfalls eine Kombination mit sämtlichen weiteren Überwachungsfunktionalitäten der vorliegenden Erfindung auf Basis eines gemeinsamen Szenarios aus der Punktewolke erfolgen.

So kann es vorteilhaft sein, wenn bei einem erfindungsgemäßen Verfahren es sich bei dem wenigstens einen absoluten Vorgabewert um den Rhythmus der Veränderung des Ortes des erkannten Objekts über die Zeit handelt. Bei einem Krampfanfall wird dieser in den meisten Fällen Zuckungen der Gliedmaßen der Person zur Folge haben. So wird die Überwachung auf die entsprechenden Körperglieder der Person durchgeführt, welche jeweils einzelne Objekte darstellen. Dabei wird ersichtlich, dass bereits die absolute Überwachung auf den tatsächlichen und absoluten Ort des jeweiligen Körpergliedes im Raum und dessen Überwachung über die Zeit eine Rhythmuserkennung in der Bewegung dieses Objekts in Form eines Körpergliedes möglich macht. Selbstverständlich kann jedoch auch zusätzlich eine Relativbetrachtung stattfinden, sodass Relativbewegungen einzelner Körperglieder zueinander ebenfalls auf rhythmische Zuckungen überwacht werden. Insbesondere kann neben der Überwachung von Teilobjekten auch eine Person im Gesamten überwacht werden, sodass durch die Punktewolke sozusagen ein Netz für diese Person aufgespannt wird, welches bei Bewegung der Person eine pulsierende Veränderung durchführt. Erfolgt dieses Pulsieren der Punktewolke für das Objekt in Form der Person bzw. des Patienten in rhythmischer Weise, so kann der entsprechende Alarm der Krampfüberwachung ausgeführt werden. Der Rhythmus für die zeitliche Veränderung des Ortes des erkannten Objekts kann als schnell und wiederkehrend beschrieben werden. Auch hier wird deutlich, dass weitere Sensoren zusätzliche Informationen für die Überwachungsfunktionalität in Form der Krampfüberwachung liefern können. Dies können z. B. Farbinformationen sein, welche die Blaufärbung der Lippen oder die Gesichtsfarbe erkennen können.

Bei der Überwachungsfunktionalität der Krampfüberwachung kann es vorteilhaft sein, wenn bei einem erfindungsgemäßen Verfahren die folgenden Schritte durchgeführt werden:
- Erkennen bzw. Bestimmen von wenigstens zwei vordefinierten Objekten in Form von benachbarten Gelenken eines Körpergliedes einer Person,
- Bestimmen des Vektors zwischen einem proximalen und einem distalen Gelenk,
- Einfügen des bestimmten Vektors in eine Zeitreihe.

Bei den voranstehenden Schritten handelt es sich um eine bevorzugte Ausführungsform für das Erzeugen einer Überwachungsfunktionalität in Form einer Krampfüberwachung. Vorzugsweise werden nicht nur ein, sondern mehrere Körperglieder der Person überwacht. Dabei kann das jeweilige Gelenk als ein Gelenkpunkt in der Punktewolke definiert werden. Die jeweiligen Körperglieder können anhand der Krampfanfälligkeit ausgewählt werden. Auch kann bei der Überwachung mehrerer Körperglieder eine Überwachung bzw. eine Definition der Art des Krampfes anhand des sich verkrampfenden bzw. sich rhythmisch bewegenden Körpergliedes erfolgen. Bei der Erkennung von Objekten wird insbesondere deren Kontur berücksichtigt, so dass eine Abdeckung durch Kleidung oder eine Bettdecke für die Funktionalität des erfindungsgemäßen Verfahrens keine entscheidende Rolle spielt.

Bei der Überwachungsfunktionalität einer Krampfüberwachung gemäß dem voranstehenden Absatz kann es weiter von Vorteil sein, wenn bei einem erfindungsgemäßen Verfahren wenigstens zwei Körperglieder überwacht werden, wobei ein Vergleich einer rhythmischen Veränderung des jeweiligen Ortes mit einer vordefinierten Mindestdauer verglichen wird. Damit wird im Falle einer Krampfsituation sichergestellt, dass es sich nicht um kurzzeitige Zuckungen des jeweiligen Körpergliedes, sondern tatsächlich um einen Krampfanfall handelt. Die Mindestdauer verhindert dementsprechend vorzugsweise einen Fehlalarm bei kurzzeitigen Zuckungen des jeweiligen Körpergliedes der Person. Dabei wird auch ein typisches Bewegungsmuster in Betracht gezogen, sodass Veränderungen des jeweiligen Zuckungsrhythmus einen Verlauf des Krampfanfalls wiedergeben können.

Eine weitere Überwachungsfunktionalität gemäß der vorliegenden Erfindung kann bei der Auswertung des Szenarios bei einer Fallüberwachung durchgeführt werden. Dabei kann sichergestellt werden, dass ein Fallen der jeweiligen Person und damit einhergehend eine entsprechende Verletzung rechtzeitig erkannt wird und dementsprechend Notfallhilfe gewährt werden kann. Dabei kann die Sicherheit der Person innerhalb des Überwachungsbereichs weiter erhöht werden.

Für das Erzeugen einer Überwachungsfunktionalität in Form einer Fallüberwachung ist es vorteilhaft, wenn bei einem erfindungsgemäßen Verfahren es sich bei dem wenigstens einen absoluten Vorgabewert um den Abstand des bestimmten Ortes des wenigstens einen erkannten Objekts, insbesondere in Form des Kopfes einer Person, von dem Fußboden und/oder einer Wand des Überwachungsbereichs handelt. Wird der Kopf als Objekt der Person erkannt, so kann dessen Abstand nicht nur zum Fußboden, sondern auch zur Wand Information darüber geben, ob sich diese Person im stehenden oder liegenden Zustand befindet. Insbesondere schnelle Veränderungen und Reduktionen des Abstandes zum Fußboden und/oder zur Wand deuten mit hoher Wahrscheinlichkeit auf einen Fall der Person hin. Damit wird durch die Korrelation zu Wandabschnitten des Überwachungsbereichs darüber hinaus ein schräger Fall gegen die Wand als Fall erkannt und dementsprechend die Sicherheit bei der Überwachungsfunktionalität einer Fallüberwachung weiter erhöht.

Ebenfalls eine mögliche Überwachungsfunktionalität bei einem erfindungsgemäßen Verfahren ist eine Funktionalität in Form eines Schwesternrufs. Darunter ist das Erkennen von Gesten zu verstehen, welche einen Alarm in Form eines Hilferufs für weiteres Dienstpersonal der medizinischen Einrichtung bzw. des Krankenhauses beinhaltet.

Bei einer Überwachungsfunktionalität für einen Schwesternruf kann es von Vorteil sein, wenn bei einem erfindungsgemäßen Verfahren es sich bei dem wenigstens einen absoluten Vorgabewert um die Veränderung des bestimmten Ortes des wenigstens einen erkannten Objekts, insbesondere in Form eines Körpergliedes einer Person, im Vergleich zu einer vordefinierten Veränderung des Ortes des wenigstens einen erkannten Objekts handelt. Mit anderen Worten wird es möglich, Gesten zu definieren und als Ortsveränderung des jeweiligen Objekts vorzugeben, welche in erfindungsgemäßer Weise einen Alarm zum Schwesternruf auslösen können. So kann beispielsweise eine Armbewegung, eine Kopfbewegung oder eine Handbewegung den entsprechenden Alarm zum Schwesternruf auslösen. Dabei ist darauf hinzuweisen, dass dies nicht nur für Patienten, sondern auch für Personal innerhalb des Überwachungsbereichs große Vorteile mit sich bringt. Wird beispielsweise eine sterile Arbeitsweise gefordert, so muss kein unsteriler Knopf mehr für den Schwesternruf gedrückt werden, sondern kann vielmehr in steriler Handlung durch eine Geste der gewünschte Alarm ausgelöst werden.

Wie bereits erläutert worden ist, können für eine erfindungsgemäße Ausführung eines Verfahrens nicht nur absolute Vorgabewerte, sondern auch relative Vorgabewerte angewendet werden. Ein relativer Vorgabewert ist dabei der Ort des erkannten Objekts mit Bezug auf den Ort eines weiteren erkannten Objekts. Selbstverständlich können Überwachungsfunktionalitäten mit relativen Vorgabewerten auch mit Überwachungsfunktionalitäten mit absoluten Vorgabewerten kombiniert werden. Auch innerhalb einer Überwachungsfunktion können relative Vorgabewerte und absolute Vorgabewerte gemeinsam einsetzbar sein.

So kann es von Vorteil sein, wenn bei einem erfindungsgemäßen Verfahren der wenigstens eine Vorgabewert für den Ort des erkannten Objekts als relativer Vorgabewert bezogen auf den Überwachungsbereich ausgebildet ist. Mit anderen Worten erfolgt das Verfahren für wenigstens zwei vordefinierte und erkannte Objekte, sodass deren Relativanordnung zueinander als Eingangswert für eine entsprechende Überwachungsfunktionalität Verwendung findet. Diese relative Überwachung kann mit anderen Überwachungsfunktionalitäten frei kombiniert werden.

Eine besonders vorteilhafte Lösung einer Überwachungsfunktionalität mit relativen Vorgabewerten ist eine sogenannte Tubusüberwachung. Diese dient dazu zu verhindern, dass Personen sich medizinische Geräte bzw. Zugänge aus dem Körper entfernen. Dies sind insbesondere Intubationsbauteile wie Beatmungstuben, Katheter und Drainagen für die Flüssigkeitsein- bzw. Ausleitung, Kompressen für das Hömostase-Management sowie Masken zur Beatmung oder zum Schutz der Augen, welche in den Körper des Patienten eingeführt sind bzw. eng anliegen. Ein Entfernen solcher medizinischer Bauteile würde mit großem Risiko einhergehen, sodass dementsprechend diese Überwachungsfunktionalität der Tubusüberwachung einen großen Sicherheitsgewinn mit sich bringt.

Um eine Tubusüberwachung zur Verfügung zu stellen, kann es vorteilhaft sein, wenn bei einem erfindungsgemäßen Verfahren es sich bei dem wenigstens einen relativen Vorgabewert um den Abstand der Orte eines ersten erkannten Objekts und wenigstens eines zweiten erkannten Objekts einer Person handelt. Hier wird gut ersichtlich, dass die Person als Objekt einzelne Teilobjekte, ebenfalls als eigene Objekte, aufweisen kann.

Beispielsweise kann der Halsbereich, in welchem ein Beatmungstubus angeordnet ist, als ein zweites Objekt definiert werden. Auch der Tubus selbst kann durch seine Verbindung zur Person bzw. zum Patienten als zweites Objekt definierbar sein. Das erste Objekt kann dabei z. B. die Hand des Patienten sein, sodass bei einer Bewegung der Hand zum Tubus dieses als Gefahr erkannt werden kann. Erreicht die Hand als Objekt den Tubus als Objekt, so besteht die Gefahr des Greifens und Herausziehens. Hier kann nun der Hauptalarm gegeben werden, wie bereits mehrfach erläutert worden ist.

Bei einer Überwachungsfunktionalität in Form einer Tubusüberwachung kann es weiter von Vorteil sein, wenn bei einem erfindungsgemäßen Verfahren es sich bei dem ersten erkannten Objekt um ein Körperglied der Person, insbesondere um eine Hand oder einen Finger, und bei dem zweiten Objekt um einen an der Person platzierten Tubus handelt. Bei dem Körperglied können selbstverständlich auch mehrere Körperglieder zusammengefasst sein, sodass alle Finger und der Handballen gemeinsam die Hand als Körperglied definieren. Insbesondere wird auf diese Weise ein Mindestabstand vorgebbar, welcher bereits ein Berühren des jeweiligen Tubus als Alarmsituation in der Überwachung definiert. Dementsprechend kann für den Tubus das Verfahren spezifisch vordefiniert durchgeführt werden. Die Erkennung der einzelnen Objekte als Tubus bzw. als Körperglied kann dabei vorzugsweise kontinuierlich überprüft werden, um Fehlüberwachungen zu vermeiden.

Vorteilhaft ist es darüber hinaus, wenn bei einer Überwachungsfunktionalität in Form einer Tubusüberwachung bei einem erfindungsgemäßen Verfahren das zweite Objekt in Form eines Tubus durch seine Abhängigkeit von wenigstens einem weiteren Objekt erkannt wird. Beispielsweise wird bei einem intubierten Patienten ein Beatmungsgerät im Überwachungsbereich angeordnet sein. Bei einem Patienten mit einem venösen oder arteriellen Zugang kann die Erkennung einer Spritzenpumpe im Überwachungsbereich erfolgen. Um die Beatmung über den Tubus sicherzustellen, ist üblicherweise ein Beatmungsschlauch vorgesehen, welcher am Tubus angeschlossen ist. Diese Informationen können als Zusatzinformationen die Lokalisierung des Tubus erlauben. So wird beim Erkennen eines Beatmungsgeräts sozusagen als Umwelteingabe grundsätzlich die Überwachungsfunktionalität der Tubusüberwachung für ein erfindungsgemäßes Verfahren aktiviert. Auch die Aktivität eines Gerätes, also ob dieses eingeschaltet ist oder ausgeschaltet, kann durch eine Umwelteingabe und damit durch Erkennung mittels des erfindungsgemäßen Verfahrens erfolgen. Auch das Vorhandensein einer weiteren Person, insbesondere medizinischen Personals kann als Umwelteingabe verwendet werden. Anschließend kann durch die Objekterkennung des Beatmungsschlauchs und dessen dreidimensionaler Verfolgung an dessen gegenüberliegendem Ende der Tubus definiert werden. Der Schnittpunkt zwischen dem Beatmungsschlauch und dem Objekt in Form des Patienten ist also der Ort des Tubus. Damit kann durch Korrelation mit weiteren Objekten ein Objekt explizit definiert werden, selbst wenn es in Form des Tubus zu klein für eine entsprechende Auflösung der Tiefenkameravorrichtung ausgebildet sein sollte. Damit wird eine erhöhte Sicherheit mit reduzierten Erkennungsqualitäten bei der Tiefenkameravorrichtung kombiniert.

Bei einer Überwachungsfunktionalität in Form einer Tubusüberwachung kann es weiter von Vorteil sein, wenn bei einem erfindungsgemäßen Verfahren für das erste Objekt ein umgebender erster Hüllkörper und für das zweite Objekt ein umgebender zweiter Hüllkörper ausgebildet werden, wobei vorzugsweise eine Kollision der beiden Hüllkörper überwacht wird. Ein Hüllkörper ist ein Körper um das jeweilige Objekt herum. Das Objekt wird dabei vorzugsweise punktförmig definiert, wie dies später noch erläutert wird. Der Hüllkörper kann dabei grundsätzlich eine freie geometrische Form aufweisen. Bevorzugt ist jedoch eine im Wesentlichen kugelförmige Ausbildung, sodass durch Vorgabe eines vordefinierten Radius als einzigem Parameter der Hüllkörper einfach und schnell definierbar wird. Ein alternativer Hüllkörper kann auch eine andere Geometrie, z.B. in Form einer Hüllbox aufweisen. Hier werden als Parameter die Länge, die Breite, die Höhe und die Rotationswinkel vorgegeben. Die Kollision der beiden Hüllkörper ist also ein Überschneiden der beiden Hüllkörper, welches als Überschreiten einer vordefinierten Grenze zu verstehen ist. Damit wird je nach Größe des jeweiligen Hüllkörpers ein rechtzeitiger Alarm möglich, sodass bereits vor Erreichen einer Berührung zwischen der Hand des Patienten und dem Tubus eine Alarmauslösung erfolgt.

Bei der Überwachungsfunktionalität in Form einer Tubusüberwachung wird ein weiterer Vorteil erzielt, wenn bei einem erfindungsgemäßen Verfahren wenigstens zwei zweite Objekte erkannt werden, wobei ein kombinierter Hüllkörper für die einzelnen Hüllkörper der zweiten Objekte ausgebildet wird. Selbstverständlich ist auch das Ausbilden von mehreren Objekten als zweiten Objekten möglich. So kann beispielsweise bei einem im Hals eingesetzten Tubus dieser als ein zweites Objekt erkannt werden. Dabei können der Kopf des Patienten und auch das Brustbein des Patienten ein weiteres zweites Objekt darstellen, sodass insgesamt drei Hüllkörper für drei zweite Objekte vordefiniert werden. Diese drei Hüllkörper können zu einem gemeinsamen Hüllkörper korreliert werden, sodass durch einfache Addition einzelner Objekte aus der Punktewolke ein komplexer Hüllkörper für dementsprechend gesteigerte Erkennungsgenauigkeit im Überwachungssystem bzw. im Überwachungsverfahren möglich wird.

Bei einer Tubusüberwachung als Überwachungsfunktionalität kann es weiter von Vorteil sein, wenn bei einem erfindungsgemäßen Verfahren der jeweilige Hüllkörper um einen Schwerpunkt des jeweiligen Objekts herum ausgebildet wird. Der Schwerpunkt ist dabei eine punktförmige Erstreckung des jeweiligen Objekts und kann als Ausgangspunkt für den Hüllkörper definiert werden. Damit wird eine einzelne und punktförmige Erstreckung des Objekts definierbar und die Realerstreckung des Objekts durch die entsprechende Größe und Ausbildung des Hüllkörpers angepasst. Die Punktdefinition des Schwerpunkts erfolgt dabei bei der Szenariobewertung bzw. bei der Szenarioerstellung in der Punktewolke.

Bei einer Überwachungsfunktionalität in Form einer Tubusüberwachung kann bei einem erfindungsgemäßen Verfahren darüber hinaus der Abstand der Orte zeitbezogen berücksichtigt werden, insbesondere bezogen auf eine Mindestdauer für die Unterschreitung eines vorgegebenen Mindestabstands. So kann eine Kollision einzelner Hüllkörper, wie sie bereits erläutert worden ist, kurzzeitig erlaubt werden, bevor erst nach einer Mindestdauer der Alarm ausgelöst wird. Auch auf diese Weise kann je nach Patientensituation die Wahrscheinlichkeit von Fehlalarmen deutlich reduziert werden.

Nachfolgend werden noch weitere mögliche Schritte eines erfindungsgemäßen Verfahrens erläutert, welche sowohl mit absoluten Vorgabewerten als auch mit relativen Vorgabewerten kombiniert werden können.

So ist es möglich, dass bei einem erfindungsgemäßen Verfahren zusätzlich zu dem wenigstens einen Vorgabewert wenigstens ein Vorstufe-Vorgabewert für den Vergleich vorgegeben und verwendet wird. Dabei handelt es sich um eine sogenannte Vorstufe zur Erzeugung eines Voralarms im Unterschied zu einem Hauptalarm. Selbstverständlich können auch mehrere Vorstufe-Vorgabewerte Verwendung finden, um entsprechende Sicherheitsabstufungen in der Alarmgebung zu erzielen. Insbesondere können unterschiedliche medizinische Personen benachrichtigt werden, in Abhängigkeit des Grades des Alarms bei der Überwachung. So kann es beispielsweise ausreichen, dass Sicherheitspersonal zu informieren oder es muss im Extremfall sogar direkt ein Mitglied des ärztlichen Personals angerufen werden.

Ein weiterer Vorteil ist erzielbar, wenn bei einem erfindungsgemäßen Verfahren der Schritt des Vergleichs wenigstens eine Funktionalität des Verfahrens aktiviert. Darunter ist die bereits mehrfach erwähnte Umwelteingabe zu verstehen. So können aus der Überwachung einzelner erkannten Objekte hinsichtlich ihres Ortes Handlungsweisen für das Verfahren abgeleitet werden. Beispielsweise kann es von Vorteil sein, wenn bei der Ausbildung einer Überwachungsfunktionalität in Form eines virtuellen Zauns das Bett hinsichtlich seiner Struktur überwacht wird. Befindet sich eine Person, welche als Patient definiert ist, innerhalb des Betts, so ist für das Einschalten der Überwachungsfunktionalität in Form des virtuellen Zauns entscheidend, dass z. B. das Bettgitter nach oben geklappt ist. Auch andere Umwelteingaben sind im Sinne der vorliegenden Erfindung möglich, um einzelne Überwachungsfunktionalitäten einzuschalten.

Ein weiterer Vorteil ist erzielbar, wenn bei einem erfindungsgemäßen Verfahren ein als Person erkanntes Objekt über den Zeitverlauf hinsichtlich ihres Ortes überwacht wird. Mit anderen Worten erfolgt ein sogenanntes Tracking dieser Person. Insbesondere wird dies für den jeweiligen Patienten durchgeführt, um für die digitale Patientenakte ein entsprechendes Bewegungsprofil nachzeichnen zu können. Selbst für den Fall, dass ein Alarm fehlläuft, kann auf diese Weise der Weg zur Alarmsituation nachgezeichnet werden. Auch ist auf Basis des Bewegungsprofils des Patienten im Überwachungsbereich eine Anpassung der Überwachungsparameter der einzelnen Überwachungsfunktionalitäten denkbar.

Ein weiterer Vorteil kann sein, wenn bei einem erfindungsgemäßen Verfahren ein Schritt einer Speicherung der bestimmten Orte bzw. von Ortsklassen (nah beim Kopfe, im Bett, am Beatmungsgerät) des wenigstens einen erkannten Objekts durchgeführt wird. Diese Speicherung wird insbesondere in Kombination mit der Ausführungsform gemäß dem voranstehenden Absatz durchgeführt, sodass bei einem Patienten als Person eine digitale Patientenakte mit der Patientenhistorie zur Verfügung gestellt wird. Dies bezieht sich natürlich insbesondere auf die Alarmereignisse während der Überwachungsphase. Auch vorteilhaft ist eine Korrelation zu anderen Alarmereignissen von weiteren Geräten, welche insbesondere direkten Patientenbezug haben. Zum Beispiel kann die Information der Bewegung des Patienten dazu genutzt werden, um bestimmte Signalartefakte als solche zu bestimmen. Hier wird gut ersichtlich wie breit ein erfindungsgemäßes Verfahren eingesetzt. So ist z.B. auch ein Einsatz in einer Patientenschleuse vor einem OP möglich, in welcher üblicherweise die Patienten alleine in ihrem Bett warten.

Ebenfalls Gegenstand der vorliegenden Erfindung ist ein Überwachungssystem, aufweisend eine Tiefenkameravorrichtung für die Überwachung eines Überwachungsbereichs und eine Kontrolleinheit. Das Überwachungssystem gemäß der vorliegenden Erfindung zeichnet sich dadurch aus, dass die Kontrolleinheit ausgebildet ist für die Durchführung eines erfindungsgemäßen Verfahrens. Dementsprechend bringt ein erfindungsgemäßes Überwachungssystem die gleichen Vorteile mit sich, wie sie ausführlich mit Bezug auf ein erfindungsgemäßes Verfahren erläutert worden sind.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Es zeigen schematisch:
- Fig. 1: eine schematische Darstellung eines Überwachungsbereichs,
- Fig. 2: die Ausführungsform der Fig. 1 in Draufsicht,
- Fig. 3: eine schematische Darstellung eines Patienten in Draufsicht,
- Fig. 4: eine weitere schematische Darstellung eines Patienten in Draufsicht,
- Fig. 5: die Darstellung gemäß Fig. 4 mit einer bewegten Situation eines Körperg liedes,
- Fig. 6: eine schematische Darstellung eines intubierten Patienten und
- Fig. 7: eine schematische Darstellung einer Punktewolke.

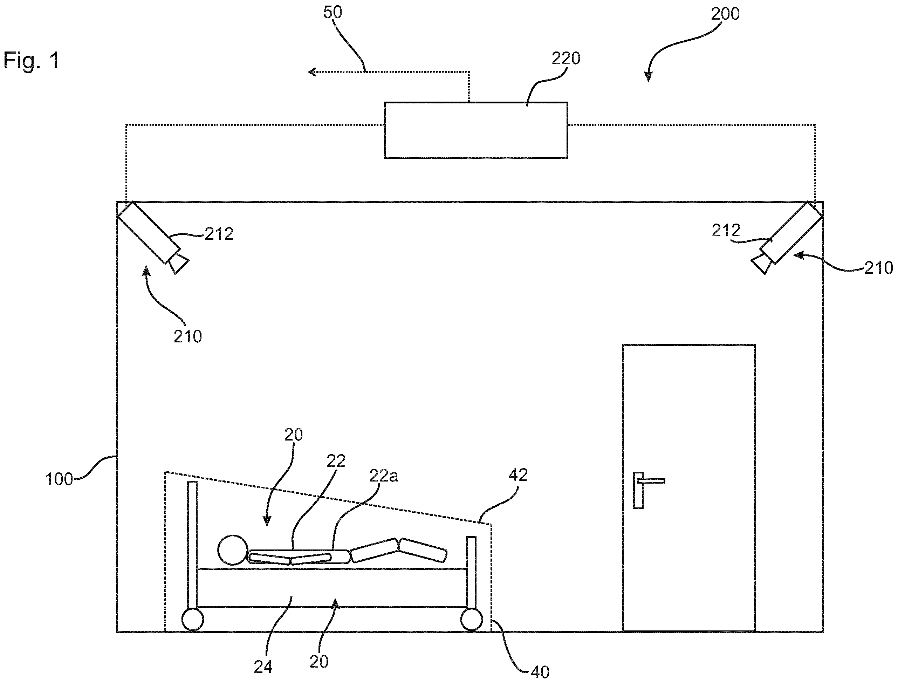

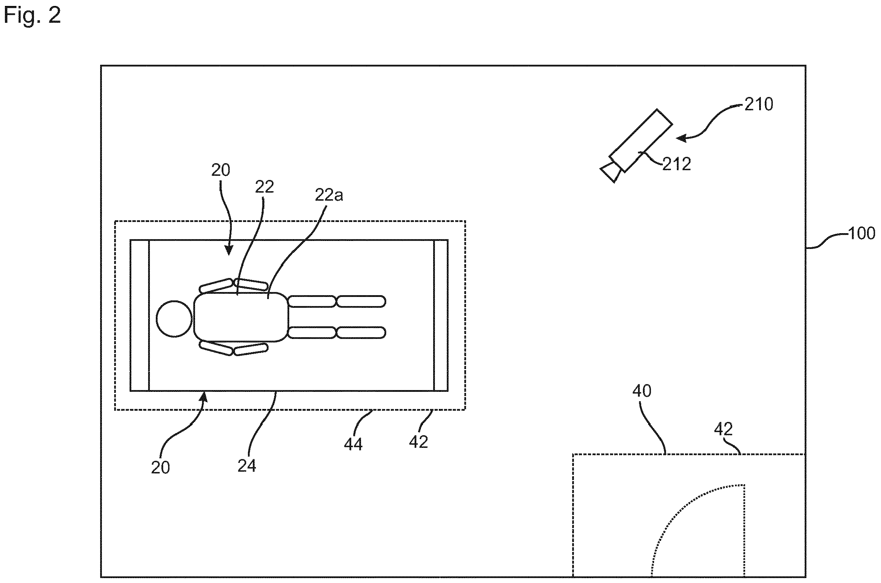

In den Fig. 1 und 2 ist schematisch ein Überwachungsbereich 100 in Form eine Patientenzimmers dargestellt. Gut zu erkennen ist ein Überwachungssystem 200 mit einer Kontrolleinheit 220 zur Ausführung eines erfindungsgemäßen Verfahrens. Das Überwachungssystem 200 ist weiter mit einer Tiefenkameravorrichtung 210 mit je zwei Tiefenkameras 212 ausgestattet. Je nach eingeschalteten Überwachungsfunktionalitäten können unterschiedlichste Alarme 50 von der Kontrolleinheit 220 abgegeben werden. Die Abgabe kann z. B. an ein Notrufsystem eines Pagers eines Arztes bzw. in einen Überwachungsraum eines Krankenhauses erfolgen. Auch eine Speicherung zu Dokumentationszwecken oder eine Weitergabe an ein IT-System des Krankenhauses ist denkbar.

Wie in Fig. 1 gut zu erkennen ist, ist innerhalb des Überwachungsbereichs 100 ein Objekt 20 in Form eines Bettes 24 angeordnet. Innerhalb des Bettes 24 befindet sich ein Objekt 20 in Form einer Person 22. Diese Person 22 ist in das Bett 24 eingestiegen bzw. wurde hineingelegt oder wurde mit dem Bett in das Zimmer gefahren und hat dabei eine Ortsgrenze 42 überschritten. Durch das Überschreiten beim Einsteigen in das Bett 24 konnte die Person 22 als Patient 22a definiert werden. Zum Status gemäß Fig. 1 befindet sich also eine Person 22, welche initialisiert als Patient 22a definiert wurde, in dem Objekt 20 in Form des Bettes 24. Dies ist als sicherer Zustand zu bezeichnen.

Die Fig. 2 zeigt in Draufsicht, dass die Ortsgrenze 42 an zwei Stellen vorgegeben sein kann. Insbesondere handelt es sich in beiden Fällen um eine sich dreidimensional erstreckende Ortsgrenze 42. Die Ortsgrenze 42 an den zwei unterschiedlichen Positionen bildet damit einen Vorgabewert 40 und einen Vorstufe-Vorgabewert 44 aus. Bewegt sich der Patient 22a aus dem Bett 24 heraus, so wird er erst die Ortsgrenze 42 des Vorstufe-Vorgabewerts 44 durchschreiten. Hierdurch kann ein Voralarm als Alarmsignal 50 von der Kontrolleinheit 220 ausgelöst werden. Bewegt sich der Patient 22a nun auf die Tür zu, so wird er zu einem bestimmten Zeitpunkt die weitere Ortsgrenze 42 in Form des Vorgabewerts 40 durchschreiten, sodass die Kontrolleinheit 220 nun den Hauptalarm als Alarmsignal 50 aussendet. Bei der hier beschriebenen Funktionalität handelt es sich um eine Überwachungsfunktion in Form eines virtuellen Zauns.

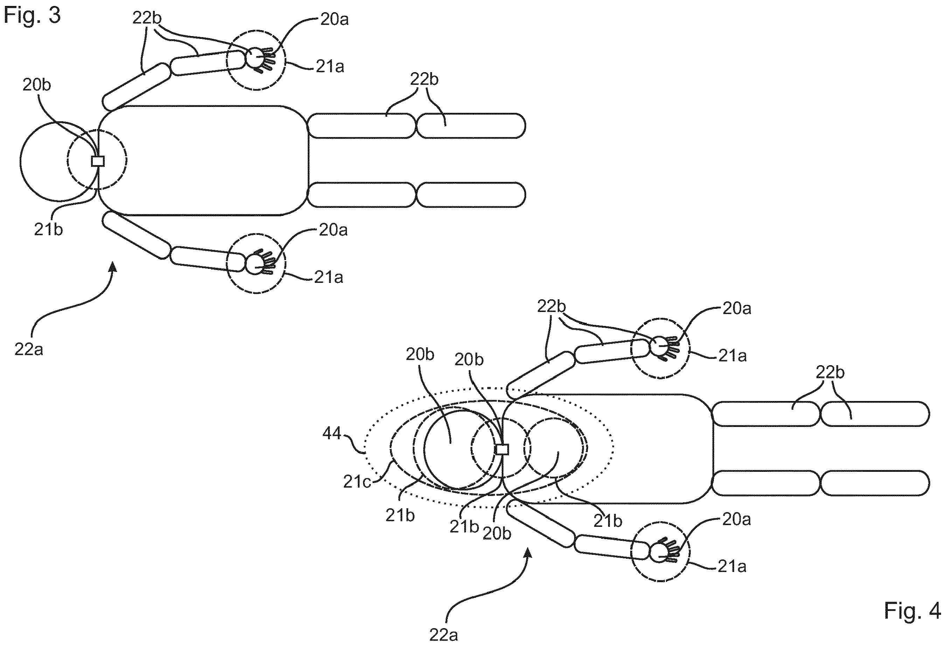

In Fig. 3 ist schematisch in Draufsicht eine ebenfalls als Patient 22a definierte Person 22 als Objekt 20 dargestellt. Dieses Objekt 20 weist eine Vielzahl von Körpergliedern 22b auf. Dies sind insbesondere die Beine, die Arme und die Hände. Dabei wird bei dieser Ausführungsform eine Überwachungsfunktionalität in Form einer Tubusüberwachung realisiert. Die dafür entscheidenden Objekte sind gemäß Fig. 3 die beiden Händen als Körperglieder 22b. Ein weiteres entscheidendes Objekt 20 ist ein zweites erkanntes Objekt 20b in Form eines Tubus am Hals des Patienten 22a. Um die beiden erkannten Objekte 20a und 20b wird jeweils ein Hüllkörper 21a und 21b in kugelförmiger Weise definiert. Der Abstand dieser jeweiligen Hüllkörper 21a und 21b definiert nun eine Unterscheidung zwischen sicherer und unsicherer Situation für den Patienten 22a.

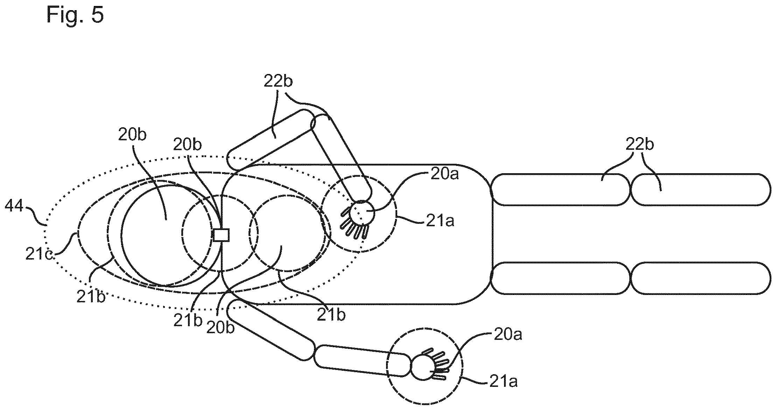

Die Figuren 4 und 5 zeigen eine komplexere Lösung für die Überwachungsfunktionalität der Tubusüberwachung. So ist hier eine Mehrzahl von zu überwachenden Objekten 20 angegeben. Zum einen sind dies als erste erkannte Objekte 20a wieder die beiden Hände des Patienten 22a mit zugehörigen ersten Hüllkörpern 21a. Weiter sind der Kopf des Patienten 22a, das Brustbein des Patienten 22a und der bereits beschriebene Tubus jeweils zweite erkannte Objekte 20b mit dementsprechend drei zugehörigen zweiten Hüllkörpern 21b. Diese lassen sich kombinieren zu einem kombinierten Hüllkörper 21c, welcher nun gegen Kollision mit den ersten Hüllkörpern 21a überwacht wird.

Der Fig. 4 ist auch eine Lösung zu entnehmen, welche die Überwachungsfunktionalität der Tubusüberwachung mit einem Vorstufe-Vorgabewert 44 versieht. Dabei handelt es sich um einen weiter vergrößerten kombinierten Hüllkörper 21 c, welcher hier als Vorstufe-Vorgabewert 44 definiert ist.

In Fig. 5 ist eine Situation dargestellt, welche bereits den Hauptalarms als Alarmsignal 50 auslöst. Dabei hat sich die linke Hand des Patienten 22a soweit in Richtung des Tubus bewegt, dass nun eine Überschreitung des Vorstufe-Vorgabewerts 44 bereits erfolgt ist. Auch dringt der erste Hüllkörper 21a bereits in den kombinierten Hüllkörper 21c ein, sodass auch der Hauptalarm als Alarmsignal 50 ausgelöst worden ist.

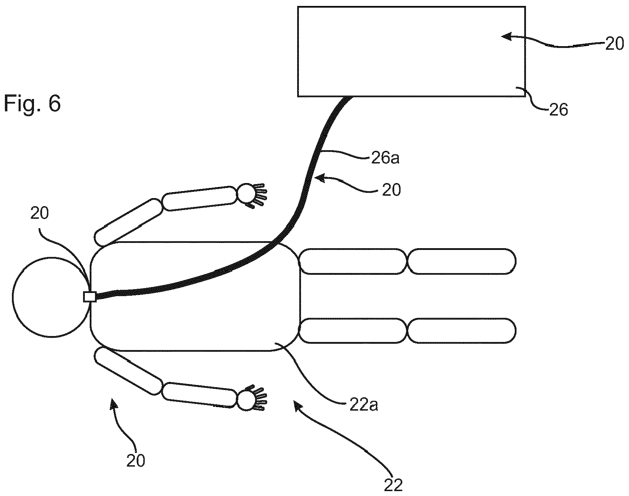

In Fig. 6 ist schematisch dargestellt, wie eine Erkennung der Position des Tubus durchgeführt werden kann. Ist beispielsweise die Auflösung der jeweiligen Tiefenkamera 212 nicht ausreichend, um den Tubus direkt zu erkennen, so kann hierfür eine Schlauchverfolgung zur Verfügung gestellt werden. So kann als Objekt 20 ein Beatmungsgerät 26 erkannt werden. Von diesem Beatmungsgerät 26 führt ein Beatmungsschlauch 26a als Objekt 20 zum Patienten 22a. Der Schnittpunkt zwischen dem Beatmungsschlauch 26a und dem Patienten 22a ist der Punkt, an welchem der Tubus platziert sein muss. Somit wird der Tubus als separates Objekt 20 über die Korrelation mit weiteren Objekten 20 erkennbar.

Durch die Fig. 6 ist auch die Möglichkeit einer beschriebenen Umwelteingabe erläuterbar. So wird durch die Erkennung eines Beatmungsgeräts 26 und eines angeschlossenen, nämlich mit dem Patienten 22a verbundenen, Beatmungsschlauchs 26a erkannt, dass es sich um eine Beatmungssituation handelt. Nun kann auf Basis dieser Information die Überwachungsfunktionalität der Tubusüberwachung eingeschaltet werden.

Die Darstellung der Fig. 3 bis 5 kann in ähnlicher Weise, mit oder ohne Hüllkörper, auch für die Erzeugung weiterer Überwachungsfunktionalitäten herangezogen werden. So können die einzelnen Körperglieder 22b der Person 22 auch Verwendung finden für die Erzeugung eines Aktivitätsindex für eine Sedierungsüberwachung. Auch kann eine Deliriumsüberwachung in dieser Weise erfolgen. Darüber hinaus kann durch Überwachung von rhythmischen Bewegungen einzelner Körperglieder 22b die Überwachungsfunktionalität der Krampfüberwachung zur Verfügung gestellt werden. Wie bereits mehrfach erläutert worden ist, wird auch hier ersichtlich, dass auf Basis eines einzelnen Szenarios unterschiedlichste Überwachungsfunktionalitäten durch unterschiedliche Auswertungsschwerpunkte erzielbar sind.

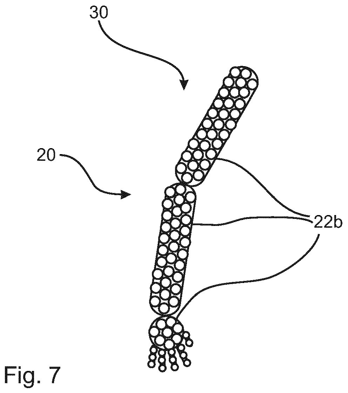

Fig. 7 zeigt schematisch eine Punktewolke 30, welche von der Tiefenkameravorrichtung 210 aufgenommen und zur Verfügung gestellt worden ist. Hier ist bereits die Korrelation bei der Auswertung, also bei der Erzeugung eines Szenarios, zu erkennen. Aus der Punktewolke 30 wurden hier Körperglieder 22b des Objekts 20 herausgebildet.

Die voranstehende Erläuterung der Ausführungsformen beschreibt die vorliegende Erfindung ausschließlich im Rahmen von Beispielen. Selbstverständlich können einzelne Merkmale der vorliegenden Erfindung, sofern technisch sinnvoll, frei miteinander kombiniert werden, ohne den Rahmen der vorliegenden Erfindung zu verlassen.

### BEZUGSZEICHENLISTE

- 20: Objekt
- 20a: erstes erkanntes Objekt
- 20b: zweites erkanntes Objekt
- 21a: erster Hüllkörper
- 21b: zweiter Hüllkörper
- 21c: kombinierter Hüllköper
- 22: Person
- 22a: Patient
- 22b: Körperglied
- 24: Bett
- 26: Beatmungsgerät
- 26a: Beatmungsschlauch
- 30: Punktewolke
- 40: Vorgabewert
- 42: Ortsgrenze
- 44: Vorstufe-Vorgabewert
- 50: Alarmsignal
- 100: Überwachungsbereich
- 200: Überwachungssystem
- 210: Tiefenkameravorrichtung
- 212: Tiefen kamera
- 220: Kontrolleinheit

## Patentansprüche

1. Verfahren für die Überwachung eines Patienten (22a) innerhalb eines medizinischen Überwachungsbereichs (100) mittels eines Überwachungssystems (200) mit einer Tiefenkameravorrichtung (210), aufweisend die folgenden Schritte:
- Erzeugen einer Punktewolke (30) des Überwachungsbereichs (100) mit dem Überwachungssystem (200),
- Auswerten der Punktewolke (30) zur Erkennung vordefinierter Objekte (20), insbesondere Personen (22),
- Bestimmen des Ortes wenigstens eines erkannten Objekts (20) im Überwachungsbereich (100),
- Vergleich des bestimmten Ortes des wenigstens einen erkannten Objekts (20) mit wenigstens einem Vorgabewert (40) für den Ort dieses erkannten Objekts (20).

2. Verfahren nach Anspruch 1, **dadurch gekennzeichnet, dass** der wenigstens eine Vorgabewert (40) für den Ort des erkannten Objekts (20) als **absoluter** Vorgabewert (40) bezogen auf den Überwachungsbereich (100) ausgebildet ist.

3. Verfahren nach Anspruch 2, **dadurch gekennzeichnet, dass** es sich bei dem wenigstens einen absoluten Vorgabewert (40) um eine Ortsgrenze (42) handelt, welche das erkannte Objekt (20), insbesondere eine als Patient (22a) definierte Person (22), nicht überschreiten darf.

4. Verfahren nach Anspruch 3, **dadurch gekennzeichnet, dass** bei einer erstmaligen Bewegung des erkannten Objekts (20) in Form einer Person (22) über die Ortsgrenze (42) diese Person (22) als Patient (22a) definiert wird.

5. Verfahren nach einem der Ansprüche 3 oder 4, **dadurch gekennzeichnet, dass** die Schritte des Verfahrens nur für den Ortsabschnitt des Überwachungsbereichs (100) auf der Seite der Ortsgrenze (42) durchgeführt werden, auf welcher sich das erkannte Objekt (20) befindet.

6. Verfahren nach einem der Ansprüche 2 bis 5, **dadurch gekennzeichnet, dass** es sich bei dem wenigstens einen absoluten Vorgabewert (40) um eine maximale und/oder minimale Veränderung des Ortes des erkannten Objekts (20) über die Zeit handelt.

7. Verfahren nach Anspruch 6, **dadurch gekennzeichnet, dass** es sich bei dem vordefinierten und erkannten Objekt (22) um wenigstens ein Körperglied (22b) einer Person (22) handelt.

8. Verfahren nach einem der Ansprüche 5 oder 7, **dadurch gekennzeichnet, dass** der absolute Vorgabewert (40) in Form der maximalen und/oder minimalen Veränderung des Ortes des erkannten Objekts (20) einstellbar ausgebildet ist.

9. Verfahren nach einem der Ansprüche 5 bis 8, **dadurch gekennzeichnet, dass** die Veränderung des Ortes des erkannten Objekts (20) über die Zeit mittels der quadratischen Abweichung des bestimmten Ortes des erkannten Objekts (20) erfolgt.

10. Verfahren nach einem der Ansprüche 2 bis 9, **dadurch gekennzeichnet, dass** es sich bei dem wenigstens einen absoluten Vorgabewert (40) um den Rhythmus der Veränderung des Ortes des erkannten Objekts (20) über die Zeit handelt.

11. Verfahren nach Anspruch 10, **dadurch gekennzeichnet, dass** dabei die folgenden Schritte durchgeführt werden:
- Erkennen von wenigstens zwei vordefinierten Objekten (20) in Form von benachbarten Gelenken eines Körpergliedes (22b) einer Person (22),
- Bestimmen des Vektors zwischen einem proximalen und einem distalen Gelenk,
- Einfügen des bestimmten Vektors in eine Zeitreihe.

12. Verfahren nach Anspruch 11, **dadurch gekennzeichnet, dass** wenigstens zwei Körperglieder (22b) überwacht werden, wobei ein Vergleich einer rhythmischen Veränderung des jeweiligen Ortes mit einer vordefinierten Mindestdauer verglichen wird.

13. Verfahren nach einem der Ansprüche 2 bis 12, **dadurch gekennzeichnet, dass** es sich bei dem wenigstens einen absoluten Vorgabewert (40) um den Abstand des bestimmten Ortes des wenigstens einen erkannten Objekts (20), insbesondere in Form des Kopfes einer Person (22), von dem Fußboden und/oder einer Wand des Überwachungsbereichs (100) handelt.

14. Verfahren nach einem der Ansprüche 2 bis 13, **dadurch gekennzeichnet, dass** es sich bei dem wenigstens einen absoluten Vorgabewert (40) um die Veränderung des bestimmten Ortes des wenigstens einen erkannten Objekts (20), insbesondere in Form eines Körpergliedes (22b) einer Person (22), im Vergleich zu einer vordefinierten Veränderung des Ortes des wenigstens einen erkannten Objekts (20) handelt.

15. Überwachungssystem (200), aufweisend eine Tiefenkameravorrichtung (210) für die Überwachung eines Überwachungsbereichs (100) und eine Kontrolleinheit (220), **dadurch gekennzeichnet, dass** die Kontrolleinheit (220) ausgebildet ist für die Durchführung eines Verfahrens mit den Merkmalen eines der Ansprüche 1 bis 14.
